# EUROPEAN PATENT APPLICATION

(11) **EP 4 840 938 A2**
(43) Date of publication of application: **18.11.2026**
(21) Application number: 26208628.3
(22) Date of filing: 21.04.2023
(51) Int. Cl.: C12N 5/00

(54) **A TISSUE CONDUCTIVE SCAFFOLDING MATERIAL**

(30) Priority: 21.04.2022 AU 2022901056
(62) Document number(s) of the earlier application(s) in accordance with Art. 76 EPC: 23790784.5 / 4 511 076
(71) Applicant: Trimph IP Pty Ltd, Sydney, NSW 2000 (AU)
(72) Inventor: Ali, FATHI, Eveleigh, New South Wales, 2015 (AU); Simin, MALEKNIA, Eveleigh, New South Wales, 2015 (AU); Terence, ABRAMS, Eveleigh, New South Wales, 2015 (AU); Will, KNOX, Eveleigh, New South Wales, 2015 (AU)
(74) Representative: M. Zardi & Co S.A.

(57) **Abstract**

Disclosed herein is a bioactive polymer for forming a tissue scaffold, the polymer comprising a first monomer for binding water, a second monomer for imparting mechanical properties to the scaffold; optionally, a third monomer for binding to a natural or synthetic peptide or protein (NSPP); and a fourth monomer for imparting phase-transition behaviour, wherein the scaffold forms a malleable structure upon hydration. Preferably, the first monomer is OEGMA; the second monomer is PLA/HEMA; the third monomer is NAS; and the fourth monomer is NIPAAm, and the polymer comprises: OEGMA in an amount of from about 1 to about 15 mol%; PLA/HEMA in an amount of from 5 to about 50 mol%; NAS in an amount of from 0 to about 15 mol%; and NIPAAm in an amount of up to about 85 mol%.

## Description

### Related Application

The present application claims convention priority from Australian provisional patent application 2022901056, filed 21 April 2022. The disclosure of AU'056 is incorporated herein by reference in its entirety.

### Field of the Invention

The present invention relates to biologically-compatible polymers, especially polymers useful in forming a tissue scaffold. In particular, the scaffold may be useful in tissue repair and regeneration.

The present invention relates to a prefabricated porous scaffold that may guide tissue integration, tissue interface regeneration and drug delivery applications. One embodiment of the invention relates to the formation of a malleable scaffold that can be administered arthroscopically or through open surgical intervention. In contact with body tissue, the scaffold is adhesive, and it is suturable which enables immobilisation of one or more tissues to support tissue regeneration and/or tissue interface integration.

In another embodiment, the present invention relates to a tissue conductive medical filler. In a further embodiment, the polymer of the present invention can form a tissue scaffold. In yet another embodiment, the scaffold forms a malleable structure upon hydration. In yet another embodiment, the scaffold can be formed *in situ* upon the increase of temperature in the body post-administration.

The present invention is envisaged to be useful in tissue engineering applications. This includes both cosmetic and therapeutic applications. Although the present invention will be described hereinafter with reference to its preferred embodiment, it will be appreciated by those of skill in the art that the spirit and scope of the invention may be embodied in many other forms.

### Background of the Invention

Any discussion of the prior art throughout the specification should in no way be considered as an admission that such prior art is widely known or forms part of common general knowledge in the field.

Tissue engineering is a biomedical engineering discipline that uses a combination of cells, engineering, materials methods, and suitable biochemical and physicochemical factors to restore, maintain, improve, or replace different types of biological tissues. Tissue engineering often involves the use of cells placed on tissue scaffolds in the formation of new viable tissue for a medical purpose but is not limited to applications involving cells and tissue scaffolds. In practice, the term is closely associated with applications that repair or replace portions of or whole tissues (*e.g*., bone, cartilage, blood vessels, bladder, skin, muscle, *etc*.). Often, the tissues involved require certain mechanical and structural properties for proper functioning. The term also relates to performing specific biochemical functions using cells within an artificially-created support system (*e.g*., an artificial pancreas, or a bio artificial liver).

Scaffolds are materials that have been engineered to cause desirable cellular interactions to contribute to the formation of new functional tissues for medical purposes. Cells are often seeded into these structures capable of supporting three-dimensional tissue formation. Scaffolds mimic the extracellular matrix of the native tissue, recapitulating the *in vivo* milieu and allowing cells to influence their own microenvironments. They usually serve at least one of the following purposes: allow cell attachment and migration; deliver and retain cells and biochemical factors; enable diffusion of vital cell nutrients and expressed products; and/or exert certain mechanical and biological influences to modify the behaviour of the cell phase.

Material selection is an essential aspect of producing a scaffold. The materials utilised can be natural or synthetic and can be biodegradable or non-biodegradable. Additionally, they must be biocompatible, meaning that they do not cause any adverse effects to cells. Silicone, for example, is a synthetic, non-biodegradable material commonly used as a drug delivery material, while gelatin is a biodegradable, natural material commonly used in cell-culture scaffolds. The best material for each application is necessarily different, and dependent on the desired mechanical properties of the material. Tissue engineering of long bone defects for example, will require a rigid scaffold with a compressive strength similar to that of cortical bone (100-150 MPa), which is much higher compared to a scaffold for skin regeneration.

There are several versatile synthetic materials used for many different scaffold applications. One of the commonly used materials is polylactic acid (PLA), a synthetic polymer. PLA is a polyester which degrades within the human body to form lactic acid, a naturally occurring chemical which is easily removed from the body. Similar materials are polyglycolic acid (PGA) and polycaprolactone (PCL). PLA is commonly combined with PGA to create poly-lactic-co-glycolic acid (PLGA). This is especially useful because the degradation of PLGA can be tailored by altering the weight percentages of PLA and PGA. This tunability, along with its biocompatibility, makes it an extremely useful material for scaffold creation.

Scaffolds may also be constructed from natural materials. Protein based materials such as collagen, or fibrin, and polysaccharidic materials- like chitosan or glycosaminoglycans (GAGs), have all proved suitable in terms of cell compatibility. Among GAGs, hyaluronic acid, possibly in combination with cross linking agents (*e.g*., glutaraldehyde, water-soluble carbodiimide, *etc*.), is a commonly-employed scaffolding material. Additionally, a fragment of an extracellular matrix protein, such as the RGD peptide, can be coupled to a non-bioactive material to promote cell attachment. Another form of scaffold is decellularised tissue, which results from the chemical extraction of cells from tissues, leaving just the extracellular matrix. This has the benefit of a fully formed matrix specific to the desired tissue type. However, a decellularised scaffold may present immune problems with future introduced cells.

All of the patents and patent publications referred to herein are incorporated by reference in their respective entireties.

WO 2013/091001 (PCT/AU2012/001566) relates to polymers, especially polymers useful as hydrogels, and to the use of hydrogels for repair or restoration of tissue. In particular, the polymers and hydrogels of WO'001 can be used for the repair or restoration of cartilage, especially articular cartilage. The polymers comprise at least a monomer for binding water, a monomer for imparting mechanical properties and a monomer for binding to an extracellular protein. The hydrogels comprise a polymer comprising at least a monomer for binding water and a monomer for binding to an extracellular protein. Crosslinking polymers by binding of the extra-cellular matrix protein forms hydrogels.

WO 2017/035587 (PCT/AU2016/050817) discloses biocompatible materials useful for tissue regeneration and repair, wherein the bioactive polymer may be in the form of a hydrogel, for example a thermoresponsive hydrogel. The bioactive polymer and resulting hydrogel of WO'587 may be used for the regeneration of bone tissue. Accordingly, the reference teaches methods of treating a bone defect in a mammal, the methods comprising administering a therapeutically effective amount of a hydrogel formed by the bioactive polymer to the mammal to treat the bone defect.

WO 2017/015703 (PCT/AU2016/050653) discloses a polymer comprising at least one antiseptic/analgesic/anti-inflammatory monomeric unit in conjunction with at least three further monomeric units, the three further monomeric units eliciting properties selected from the group consisting of: temperature activation, water solubility, mechanical strength, protein/polysaccharide bonding capacity, and combinations thereof. In particular, WO'703 discloses a polymer, wherein the water-soluble monomeric unit is a hydrophilic ethylene glycol (OEGMA) moiety; the mechanical strength-conferring monomeric unit is polylactide-co-2-hydroxy-ethylmethyl acrylate (PLA/HEMA); the protein-reactive monomeric unit is an N-acryloxysuccinimide (NAS) moiety; and the thermosetting monomeric unit is an N- isopropyl acrylamide (NIPAAm) moiety. The antiseptic/analgesic/anti-inflammatory monomeric unit comprises a methacrylic ester derivative of salicylic acid (5-HMA or 4-HMA, or a combination thereof).

WO 2021/119727 (PCT/AU2020/051332) teaches a composition comprising a polymer and a natural or synthetic peptide or protein (NSPP) as Thymosin beta-4. The polymer comprises a first monomer for binding water, a second monomer for imparting mechanical properties, a third monomer for binding to an NSPP and a fourth monomer for imparting phase-transition behaviour. In particular, the composition forms an adhesive and flowable hydrogel upon administration into the body or onto the body surface, thereby assists in tissue repair and regeneration. Accordingly, WO'727 discloses methods of tissue repair and/or regeneration, the methods comprising administering the compositions by injection or by administering an aerosol, thereby to form a hydrogel at the body temperature of a mammal.

All four applications referenced above are assigned to the present Applicant, Trimph IP Pty Ltd, of Sydney, Australia.

It is an object of the present invention to overcome or ameliorate at least one of the disadvantages of the prior art, or to provide a useful alternative.

There is a general need in the art for new scaffolds for tissue repair and/or regeneration. There is a general need in the art for new scaffolds to allow tissue interface regeneration and integration. There is a general need in the art for new scaffolds that allows tissue ingrowth as opposed to superficial tissue on-growth. There is a general need for scaffolds for tissue repair and/or regeneration that negate the criticality of water solubility. There is a general need for tissue scaffolds that can be manufactured by using different processing methods. There is a general need for tissue scaffolds that can be tuned to alter their micro-environment to address the requirements. There is a general need for tissue scaffolds that can bind to peptide and/or protein. There is a general need for tissue scaffolds that can bind to different hydrophilic and hydrophobic drugs. There is a general need for tissue scaffolds that are non-adhesive to gloves for effective manipulation. There is a general need for tissue scaffolds that are malleable, which allow effective delivery to the site, arthroscopically or via open surgical intervention. There is a need for tissue scaffolds that are adhesive to the treatment site. There is a general need for tissue scaffolds that are suturable (or stapled) for internal fixation.

It is against this background that the present invention has been developed. Various embodiments of the present invention may find utility relating to one or more of the general needs identified above.

In particular, the present invention is useful for soft tissue applications, such as to skin grafting to immobilise the grafter tissue, preparing the site for future skin grafting, tendon and/or ligament healing and/or augmentation and/or reinforcement and/or stabilisation, repair of partial or full thickness rotator cuff tears (including but not limited to superior capsular reconstruction (SCR)) soft tissue healing in shoulder arthroplasty, knee arthroplasty and hip arthroplasty, cruciate ligament (including anterior, posterior and medial cruciate ligament, ACL, PCL, MCL) and/or other ligament/tendon tears (including but not limited to Achilles ligament, biceps tendons, patella tendons) and hard tissue applications. The present invention may be also useful for drug delivery applications.

Although the invention will be described with reference to specific examples it will be appreciated by those skilled in the art that the invention may be embodied in many other forms.

### Summary of the Invention

In a broad form, the present invention is embodied as a filler, wherein upon hydration the filler forms a malleable scaffold. The scaffold is well-tolerated in the body with minimal inflammatory response and can be used as a generic matrix to support tissue healing and integration of two or more tissue interfaces. The scaffold is host tissue-conductive but not inductive . The filler can be used intraoperatively to be hydrated with saline, a patient's own blood, platelet reach plasma (PRP), platelet reach fibrin, bone marrow aspirate, and/or other blood/cell/tissue products and/or extracts (from autologous and allogenic sources) to form malleable scaffolds. The resulting scaffold can be administered arthroscopically or through open surgical intervention. In contact with body tissue, the scaffold is adhesive, and it is amenable to suturing and surgical stapling which enables immobilisation of one or more tissues to support tissue regeneration and/or tissue interface integration.

According to a first aspect of the invention there is provided a polymer for forming a tissue scaffold, the polymer comprising:
a first monomer for binding water;
a second monomer for imparting mechanical properties to the scaffold;
optionally, a third monomer for binding to a natural or synthetic peptide or protein (NSPP); and
a fourth monomer for imparting phase-transition behaviour;
wherein the scaffold forms a malleable structure upon hydration.

In an embodiment, the first monomer is selected from: polyethers, polyvinyl alcohol (PVA); poly(vinyl pyrrolidone) (PVP); poly(amino acids) and dextran.

In an embodiment, the polyethers are selected from: polyethylene glycol (PEG), oligo(ethylene glycol) (OEG), polyethylene oxide (PEO), polyethylene oxide-co-propylene oxide (PPO), co-polyethylene oxide block or random copolymers thereof.

In an embodiment, the first monomer is oligo (ethylene) glycol monomethyl ether methacrylate (OEGMA).

In an embodiment, the second monomer is a methacrylate, or a random co-polymer comprising a methacrylate.

In an embodiment, the second monomer is selected from: hydroxyethyl methacrylate (HEMA), a hydroxyethyl methacrylate poly(lactic acid) copolymer (PLA/HEMA), poly(lactic acid), poly( caprolactone ), poly(glycolide ), poly(glycolide-colactide) or poly(glycolide-co-caprolactone).

In an embodiment, the second monomer is hydroxyethyl methacrylate poly(lactic acid) (PLA/HEMA).

In an embodiment, the third monomer has electrophilic functional groups for binding to the NSPP.

In an embodiment, the third monomer is selected from: N-hydroxysulfosuccinimide (SNHS), N-hydroxyethoxylated succinimide (ENHS), and N-acryloxysuccinimide (NAS).

In an embodiment, the third monomer is N-acryloxysuccinimide (NAS).

In an embodiment, the fourth monomer has a lower critical solution temperature (LCST) less than about 37 °C.

In an embodiment, the fourth monomer is selected from: poly(ethylene oxide)/poly(propylene oxide) and poly(N-isopropylacrylamide) (PNIPAAm) homopolymers and copolymers.

In an embodiment, the fourth monomer is (N-isopropylacrylamide) (NIPAAm).

In an embodiment, the polymer comprises the first monomer in an amount of from about 1 to about 15 mol%.

In an embodiment, the polymer comprises the second monomer in an amount of from about 5 to about 50 mol%.

In an embodiment, the polymer comprises the third monomer in an amount of from about 0 to about 15 mol%.

In an embodiment, the polymer comprises the fourth monomer in an amount of from about 50 to about 85 mol%.

In an embodiment, the polymer comprises: the first monomer in an amount of from about 1 to about 15 mol%; the second monomer in an amount of from about 5 to about 50 mol%; the third monomer in an amount of 0 to about 15 mol%; and the fourth monomer in an amount which makes up the remainder to 100% of the polymer.

In an embodiment, the first monomer is OEGMA; the second monomer is PLA/HEMA; the third monomer is NAS; and the fourth monomer is NIPAAm, wherein the polymer comprises: OEGMA in an amount of from about 1 to about 15 mol%; PLA/HEMA in an amount of from 5 to about 50 mol%; NAS in an amount of from 0 to about 15 mol%; and NIPAAm in an amount of up to about 85 mol%.

According to a second aspect of the present invention there is provided a tissue scaffold comprising a polymer as defined according to the first aspect of the present invention, wherein the scaffold forms a malleable structure upon hydration.

In an embodiment, the scaffold is hydrated intraoperatively. In another embodiment, the scaffold is hydrated with saline, aqueous solutions, autologous or allogenic blood, cell or tissue products, or a combination thereof.

In an embodiment, the scaffold is hydrated with a patient's own blood, platelet reach plasma (PRP), bone marrow aspirate, platelet reach fibrin, or other blood or tissue derived products or a combination thereof.

In an embodiment, the scaffold is hydrated with allogenic blood, platelet reach plasma (PRP), platelet reach fibrin, or other blood or tissue derived products or a combination thereof.

In an embodiment, the scaffold is non-adhesive to surgical gloves. In an embodiment, the scaffold is adhesive to a treatment site.

In an embodiment, the scaffold has no tissue-inductive properties.

In an embodiment, the scaffold is formed following administration to a mammal.

In an embodiment, wherein the scaffold is formed at body temperature.

In an embodiment, the administration is by injection or spray.

According to a third aspect of the present invention there is provided a malleable structure formed from a tissue scaffold as defined according to the second aspect of the present invention.

According to a fourth aspect of the present invention there is provided a method of making a tissue scaffold, the method comprising the steps of:
dissolving an aqueous solution of a polymer according to the first aspect of the present invention in a buffer solution to obtain a resulting solution; and
freeze-drying the resulting solution;
wherein the scaffold has a morphology alterable by adjusting freeze-drying parameters and concentration of the polymer.

In an embodiment, at least one drug or biological moiety is added to the resulting solution between the dissolving and freeze-drying steps.

In an embodiment, the drug or biological moiety is selected from: antibiotics, growth factors, live viruses, or a combination thereof.

According to a fifth aspect of the present invention there is provided a method of making a tissue scaffold, the method comprising the steps of:
dissolving a polymer according to the first aspect of the invention in an organic solvent to obtain a solution;
precipitating the solution in water as an anti-solvent to obtain a gel; and
freeze-drying the gel.
wherein the scaffold has a morphology alterable by adjusting freeze-drying parameters.

In an embodiment, the organic solvent is miscible in water.

According to a sixth aspect of the present invention there is provided a method of making a tissue scaffold, the method comprising the steps of:
dissolving the polymer according to the first aspect of the invention in an organic solvent to obtain a solution; and
electrospinning the solution.

According to a seventh aspect of the present invention there is provided a method of making a tissue scaffold, the method comprising:
dissolving the polymer as defined according to the first aspect of the invention in aqueous solutions, autologous blood, cell or tissue products, or a combination thereof;
wherein the scaffold is injectable or sprayable.

According to an eighth aspect of the present invention there is provided use of a polymer according to the first aspect of the invention in the manufacture of a tissue scaffold for skin grafting to immobilise the grafter tissue, preparing the site for future skin grafting, tendon and/or ligament healing and/or augmentation and/or reinforcement and/or stabilisation, repair of partial or full thickness rotator cuff tears, soft tissue healing in shoulder arthroplasty, knee arthroplasty and hip arthroplasty, cruciate ligament and/or other ligament/ tendon tears.

According to a ninth aspect of the present invention there is provided use of a tissue scaffold according to the second aspect of the invention for the repair and/or regeneration of tissue.

According to a tenth aspect of the present invention there is provided a method for repair and/or regeneration of tissue; supporting skin grafting to immobilise the grafter tissue; preparing the site for future skin grafting; tendon and/or ligament healing and/or augmentation and/or reinforcement and/or stabilisation; repair of partial or full thickness rotator cuff tears; soft tissue healing in shoulder arthroplasty, knee arthroplasty and hip arthroplasty, cruciate ligament and/or other ligament/ tendon, the method comprising administering to a mammal a tissue scaffold according to the second aspect of the invention.

In an embodiment, the administration is by injection or spray. In an embodiment, the scaffold is administered arthroscopically or through open surgical intervention. In an embodiment, the scaffold is hydrated to form a malleable structure upon intraoperative administration.

According to a eleventh aspect of the present invention there is provided a tissue scaffold according to the second aspect of the invention, for use in repair and/or regeneration of tissue; supporting skin grafting to immobilise the grafter tissue; preparing the site for future skin grafting; tendon and/or ligament healing and/or augmentation and/or reinforcement and/or stabilisation; repair of partial or full thickness rotator cuff tears; soft tissue healing in shoulder arthroplasty, knee arthroplasty and hip arthroplasty, cruciate ligament and/or other ligament/ tendon tears.

In an embodiment, the scaffold is administered by injection or spray. In an embodiment, the scaffold is administered arthroscopically or through open surgical intervention. In an embodiment, the scaffold is hydrated to form a malleable structure upon intraoperative administration.

According to a twelfth aspect of the present invention there is provided a kit for forming a tissue scaffold, the kit comprising a polymer, wherein the polymer comprises: a first monomer for binding water; a second monomer for imparting mechanical properties to the scaffold; optionally, a third monomer for binding to a natural or synthetic peptide or protein (NSPP); and a fourth monomer for imparting phase-transition behaviour, wherein the scaffold forms a malleable structure upon hydration.

### Definitions and Nomenclature

In describing and claiming the present invention, the following terminology will be used in accordance with the definitions set out below. It is also to be understood that the terminology used herein is for the purpose of describing particular embodiments of the invention only and is not intended to be limiting. Unless defined otherwise, all technical and scientific terms used herein have the same meaning as commonly understood by one having ordinary skill in the art to which the invention pertains.

Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The terms "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the invention.

As used herein a wording defining the limits of a range or length such as, for example, "from 1 to 5" means any integer from 1 to 5, *i.e.,* 1, 2, 3, 4 and 5. In other words, any range defined by two integers explicitly mentioned is meant to comprise and disclose any integer defining the limits and any integer comprised in the range.

Other than in the operating examples, or where otherwise indicated, all numbers expressing quantities of ingredients or reaction conditions used herein are to be understood as modified in all instances by the term "about". The examples are not intended to limit the scope of the invention. In what follows, or where otherwise indicated, "%" will mean "weight %", "ratio" will mean "weight ratio" and "parts" will mean "weight parts".

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard variations found in their respective testing measurements.

The present specification uses the following abbreviations:
- ECM: Extracellular matrix
- EHNS: N-hydroxyethoxylated succinimide
- HEMA: Hydroxyethyl methacrylate
- LA: Lactic acid
- NAS: N-acryloxysuccinimide
- NIPAAm: N-isopropylacrylamide
- NSPP: Natural or synthetic peptide or protein
- OEG: Oligo(ethylene glycol)
- OEGMA: Oligo (ethylene) glycol monomethyl ether methacrylate
- PBS: Phosphate-buffered saline
- PEG: Polyethylene glycol
- PEO: Polyethylene oxide
- PLA: Poly(lactic acid)
- PLA/HEMA: Hydroxyethyl methacrylate poly(lactic acid)
- PPO: Polyethylene oxide-*co*-propylene oxide
- PVA: Polyvinyl alcohol
- PVP: Poly(vinyl pyrrolidone)
- PNPHO: Poly(N-isopropylacrylamide-*co*-(N-acryloxysuccinimide)-*co-*(polylactide/2-hydroxy methacrylate)-*co*-(oligo (ethylene glycol) / Poly(NIPAAm-*co*-NAS-*co*-(PLA/HEMA)-*co*-OEGMA)
- PPHO: Poly(N-isopropylacrylamide-*co*-(polylactide/2-hydroxy methacrylate)-*co-*(oligo (ethylene glycol) / Poly(NIPAAm-*co*-(PLA/HEMA)-*co*-OEGMA)
- SNHS: N-hydroxysulfosuccinimide
- TB4: Thymosin beta-4 or Thymosin β-4

### Brief Description of the Drawings

Preferred embodiments of the invention will now be described, by way of example only, with reference to the accompanying drawings in which:
Figure 1(a) shows the surface of a PNPHO scaffold formed from a polymer of the present invention and Figures 1(b) to 1(d) show its morphology at 100, 50 and 20 micron resolution, respectively.
Figure 2 shows the PNPHO scaffold handling after hydration, illustrating the structural stability of the product during handling for effective manipulation and surgical administration. Figure 2 shows sequential steps in defolding/unfolding the scaffold.
Figure 3(a) shows an adhesion test of the PNPHO scaffold onto a soft tissue (epidermis).
Figure 3(b) shows a peel test of the PNPHO scaffold from a soft tissue (epidermis).
Figure 4(a) shows an adhesion test of the PNPHO scaffold onto a hard surface (simulated hard tissue, *e.g*., bone).
Figure 4(b) shows a peel test of the PNPHO scaffold onto a hard surface (simulated hard tissue, *e.g*., bone).
Figure 5(a) shows the ¹H NMR spectrum of PPHO (NAS = 0 mol%) in CD₃CN. The presence of characteristic monomeric peaks confirmed the formulation of PPHO.
Figure 5(b) shows the comparative ¹H NMR spectra of PNPHO and PPHO; (a) ¹H NMR spectrum of PPHO (NAS = 0 mol%), the presence of characteristic peaks confirmed the formulation of PPHO (NIPAAm = ~90 mol%, PLA/HEMA = ~10 mol% and OEGMA = ~1 mol%) and (b) ¹H NMR spectrum of PNPHO (NAS = 10 mol%, OEGMA = 5 mol%). The PPHO ¹H NMR spectrum was collected with CD₃CN and the PNPHO ¹H NMR spectrum was collected with CDCl₃. The necessary change in solvent for NMR data acquisition was due to the hydrophobicity of PPHO which prevents its dissolution in CDCl₃.
Figure 5(c) shows HPLC chromatograms at 230 nm showing PNPHO eluting at 6.2 min and PPHO at 7.2 min. The difference in the elution time between confirms that PPHO elutes at higher percentage of acetonitrile compared to PNPHO and further confirms the hydrophobicity of PPHO in comparison with PNPHO.
Figure 6(a) shows the PPHO adhesion on soft tissue and its adhesivity despite its inherent hydrophobic properties.
Figure 6(b) shows the PPHO adhesion on a hard surface to display the adhesivity of the product the product on simulated hard tissue.
Figure 7 shows the body weight changes in mice implanted with the PNPHO and/or PPHO scaffolds over a period of 42 days (time point = days post-implantation).

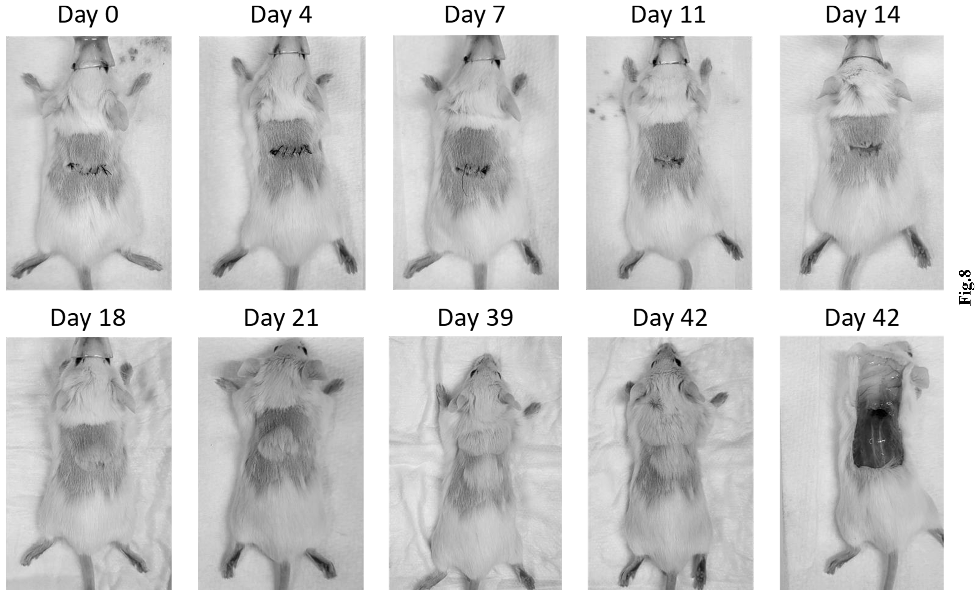

Figure 8 shows digital images of macroscopic observations at various time points over 42 days. Note that any fur regrowth on the dorsum of the mice is not correlated with scaffold presence. This is the normal regrowth pattern and was generally consistent in all mice.
Figure 9 shows digital images of mice sacrificed on day 7, day 14, day 21 and day 42 post-operatively, respectively.
Figure 10 shows an H&E image of skin histology of mouse ID "S-4" in week 1.
Figure 11 shows an H&E image of skin implantation sites in weeks 1, 2, 3 and 6 post-operatively.

### Detailed Description of the Embodiments

The present invention will now be more fully described with reference to the accompanying examples and drawings. It should be understood, however, that the description following is illustrative only and should not be taken in any way as a restriction on the generality of the invention described above.

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

One skilled in the art will recognise many methods and materials similar or equivalent to those described herein, which could be used in the practice of the present invention. The present invention is in no way limited to the methods and materials described.

It will be understood that the invention disclosed and defined in this specification extends to all alternative combinations of two or more of the individual features mentioned or evident from the text or drawings. All of these different combinations constitute various alternative aspects of the invention.

Disclosed broadly herein is the use of a polymer that is tailored for tissue. A scaffold formed from the polymer preferably forms a malleable structure upon hydration.

### Polymers

The term "polymer", as used herein, refers to a large molecule (macromolecule) composed of repeating structural units (monomers). These subunits are typically connected by covalent chemical bonds. Polymers can be linear or branched polymers. Preferably, the polymers of the present invention are copolymers comprising three or more different monomers. For example, in one embodiment, the polymer of the present invention includes a first water-binding monomer, a second monomer that is capable of imparting mechanical properties to a tissue scaffold, and a third monomer that has a functional group for binding to an NSPP.

The term "monomer", as used herein, refers to a structural unit that can be combined to form a polymer, but that itself may also be a polymer, or a derivative of a monomer or polymer. Monomers of this type are herein also referred to as "macromonomers". Herein a "macromonomer" is a polymer or oligomer the molecules of which each have one end-group that acts as a monomeric molecule, so that each polymer or oligomer molecule contributes only a single monomer unit to a chain of the product polymer.

The polymer of the present invention comprises: a first monomer for binding water; a second monomer for imparting mechanical properties to the tissue scaffold; an optional third monomer for binding to a natural or synthetic peptide or protein (NSPP); and a fourth monomer for imparting phase-transition behaviour.

### First monomer: Water-binding monomer

As discussed above, the advantages of the tissue scaffolds of the present invention can be attributed, at least in part, to the particular components that make up the polymers of the present invention. A particularly advantageous property of the polymers of the present invention is their water-binding capacity. The presence of water in the scaffolds of the present invention provides an environment that resembles both that of the natural environment of the damaged tissue (which assists in tissue regeneration) and the required compression resistance to the scaffolds.

Accordingly, the preferred polymers used herein should include monomers or units that are able to bind water to such a capacity that a malleable structure is able to form when the polymer is hydrated. In addition, the structure thus formed should have the required compression resistance and resilience.

A person skilled in the art will understand that water-binding monomers need to be present in the polymers of the present invention in proportions that are sufficient to produce a polymer that fulfils these requirements. Generally, the proportion of water-binding monomers in the polymer is about 3:1, about 2:1, about 1:1, about 1:2, about 1:3, about 1:4, about 1:5, about 1:10, about 1:20, about 1:30, about 1:40, about 1:50 molar ratio of water binding:mechanical strength monomers. In fact, the water-binding monomers need to make the polymer not only hydrophilic, but impart much more significant water-binding capacities to the polymer. Accordingly, polymers in accordance with the present invention will have water-binding capacities of between about 70% and about 500%, between about 80% and about 400%, between about 90% and 300% or between about 100% and 200%. For example, the water-binding capacity of the polymers of the present invention is about 70%, about 80%, about 90%, about 100%, about 110%, about 120%, about 130%, about 140%, about 150%, about 160%, about 170%, about 180%, about 190%, about 200%, about 210%, about 220%, about 230%, about 240%, about 250%, about 260%, about 270%, about 280%, about 290%, about 300%, about 310%, about 320%, about 330%, about 340%, about 350%, about 360%, about 370%, about 380%, about 390%, about 400%, about 410%, about 420%, about 430%, about 440%, about 450%, about 460%, about 470%, about 480%, about 490%, or about 500%.

Suitable examples of water-binding monomers include those that can be synthesised into polymers such as polyethers (*e.g*., alkylene polyoxides such as polyethylene glycol (PEG), oligo(ethylene glycol) (OEG), polyethylene oxide (PEO), polyethylene oxide-co-propylene oxide (PPO), co-polyethylene oxide block or random copolymers, polyvinyl alcohol (PVA)), poly(vinyl pyrrolidinone) (PVP), poly(amino acids) and dextran. The polyethers, and more particularly oligo(oxyalkylenes) (*e.g*., OEG), are especially preferred, because they have the requisite water-binding capacity, are simple to synthesise and/or purchase, and are inert, in the sense that they illicit minimal or no immune response from the tissues into which they are placed.

In addition, any of a variety of hydrophilic functionalities can be used to make a monomer (and therefore a polymer formed from such a monomer) water soluble. For example, functional groups like phosphate, sulphate, quaternary amine, hydroxyl, amine, sulfonate and carboxylate, which are water soluble, may be incorporated into a monomer to make it water soluble.

Monomers may also be reacted with other compounds to form "macromonomers". Thus, the first monomer may optionally be a macromonomer.

A preferred first monomer which is a macromonomer is oligo(ethyleneglycol) monomethyl ether methacrylate (OEGMA), which is a hydrophilic monomer composed of two hydrophilic monomers: ethylene glycol and methacrylate.

Preferably, the polymer comprises the first monomer in an amount of from about 1 to about 15 mol%. In various embodiments, the first monomer may be present in about 1, about 2, about 3, about 4, about 5, about 6, about 7, about 8, about 9, about 10, about 11, about 12, about 13, about 14, or about 15 mol%. In various embodiments, the first monomer may be present from about 1 to about 15, about 2 to about 14, about 3 to about 13, about 4 to about 12, about 5 to about 11, about 6 to about 10, about 7 to about 9, or about 8 mol%.

### Second monomer: Monomer imparting mechanical properties

As discussed above, the advantageous properties of the tissue scaffolds of the present invention can be attributed, in part, to the particular components that make up the polymers of the present invention. In some embodiments, the polymers of the present invention are able to contribute additional mechanical properties and adhesivity to the scaffolds of the present invention.

A person skilled in the art will understand that monomers capable of imparting mechanical properties to a tissue scaffold need to be present in the polymers of the present invention in proportions that are sufficient to produce a tissue scaffold having the desired mechanical properties. Generally, the proportion of "mechanical" monomers in the polymer is about 3:1, about 2:1, about 1:1, about 1:2, about 1:3, about 1:4, about 1:5, about 1:10, about 1:20, about 1:30, about 1:40, about 1:50 molar ratio of water binding:mechanical strength monomers. Suitable examples of monomers that are capable of imparting mechanical properties (e.g. compression resistance) to a scaffold include acrylates such as hydroxyethyl methacrylate (HEMA), polyesters such as poly(lactic acid), poly(caprolactone), poly(glycolide), and their random co-polymers (e.g. poly(glycolide-*co*-lactide) and poly(glycolide-*co*-caprolactone)).

Monomers may also be reacted with other compounds to form "macromonomers". A preferred second monomer which is a macromonomer is hydroxyethyl methacrylate poly(lactic acid) (PLA/HEMA).

Preferably, the polymer comprises the second monomer in an amount of from about 1 to about 50 mol%. In various embodiments, the second monomer may be present in about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50 mol%. In various embodiments, the second monomer may be present from about 1 to about 15, about 2 to about 49, about 3 to about 48, about 4 to about 47, about 5 to about 46, about 6 to about 45, about 7 to about 44, about 8 to about 43, about 9 to about 42, about 10 to about 41, about 11 to about 40, about 12 to about 39, about 13 to about 38, about 14 to about 37, about 15 to about 36, about 16 to about 35, about 17 to about 34, about 18 to about 33, about 19 to about 34, about 20 to about 33, about 21 to about 30, about 22 to about 29, about 23 to about 28, about 24 to about 27, or about 25 to about 26 mol%.

A person skilled in the art would understand that the amount of the second monomer occupies a broader range than the other monomers as mechanical strength and adhesivity are the critical factors in the present invention.

### Third monomer: NSPP-binding monomer

As discussed above, the tissue scaffolds used in the present invention can optionally be formed by combining the polymer with an NSPP. In order to effectively combine the polymer with the NSPP, preferably monomers or units that have a crosslinking ability are included in the polymer.

This crosslinking ability means that the polymers are able to bind to NSPPs and, by doing so, crosslink the NSPP to form scaffolds containing the NSPP. Alternatively, via a similar mechanism, the NSPPs act as the crosslinker, thereby crosslinking the polymer to form a scaffold.

In order to produce a polymer that is capable of binding to NSPPs, a person skilled in the art will understand that monomers capable of binding to an NSPP need to be present in the polymers of the present invention in proportions that are sufficient to crosslink with an NSPP, such that a tissue scaffold can be formed in the presence of water. Generally, the proportion of "crosslinking" monomers in the polymer is at about 15:1, about 10:1, about 5:1, about 4:1, about 3:1, about 2:1, about 1:1, about 1:2, about 1:3, about 1:4, about 1:5, about 1:10, about 1:15 of crosslinking monomer:water binding monomer.

Monomers that are capable of binding to NSPPs generally have either electrophilic or nucleophilic functional groups, such that a nucleophilic functional group on, for example, an NSPP may react with an electrophilic functional group on the monomer, to form a covalent bond.

Therefore, for example, if an NSPP has nucleophilic functional groups such as amines, the polymer may have electrophilic functional groups such as N-hydroxysuccinimides (NHS). Other electrophilic functional groups that are suitable for use in the present invention are N-hydroxysulfosuccinimide (SNHS) and N-hydroxyethoxylated succinimide (ENHS). An example of a monomer of this type is N-acryloxysuccinimide (NAS). On the other hand, if an NSPP has electrophilic functional groups, then the polymer may have nucleophilic functional groups such as amines or thiols.

Preferably, the polymer comprises the third monomer in an amount of up to 15 mol%. In various embodiments, the third monomer may be present in about 0, about 1, about 2, about 3, about 4, about 5, about 6, about 7, about 8, about 9, about 10, about 11, about 12, about 13, about 14, or about 15 mol%. In various embodiments, the third monomer may be present from about 0 to about 1, about 1 to about 15, about 2 to about 14, about 3 to about 13, about 4 to about 12, about 5 to about 11, about 6 to about 10, about 7 to about 9, or about 8 mol%.

A skilled person would understand that the polymer may be formed from hydrophobic compositions, therefore the third monomer is optional in the polymer.

### Fourth monomer: Phase-transition monomer

In another embodiment of the present invention, the polymer may further include a fourth monomer that is capable of imparting phase transition characteristics to the scaffold, thereby ensuring post-administration stability of the scaffold. Further, these phase-transition characteristics allow the polymers of the present invention to form scaffolds, of which various properties (such as viscosity) can be varied by altering factors such as pH and temperature. The scaffolds are designed such that the lower critical solution temperature (LCST) is below body temperature. Various thermo-responsive and injectable polymers including poly(ethylene oxide)/poly(propylene oxide) and poly(N-isopropylacrylamide) (PNIPAAm) copolymers are suitable for use in the present invention.

Generally, the proportion of phase-transition monomers in the polymer is at least about 3:1 molar ratio of phase-transition monomer:water binding monomer. This ratio can increase to, for example, about 10:1, about 11:1, about 12:1, about 13:1, about 14:1, about 15:1, about 16:1, about 17:1, about 18:1, about 19:1, about 20:1, about 25:1, about 30:1, about 35:1, about 40:1, about 45:1, about 50:1, about 55:1, about 60:1, about 65:1, about 70:1 molar ratio, about 75:1, about 80:1 and about 85:1 of phase-transition monomer:water binding monomer.

Preferably, the polymer comprises the fourth monomer in an amount which makes up the remainder to 100% of the polymer composition. In an embodiment, the mol% of the fourth monomer can be up to about 85%, preferably, about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84 or 85 mol%.

### Other polymer properties

It will be understood by a person skilled in the art that, by combining different types of monomers, polymers can be produced that have a range of different properties. In addition, by incorporating particular monomers or functional groups into a pre-existing polymer, the properties of the polymer can be modified. For example, co-polymerisation of HEMA monomers with other monomers (such as methyl methacrylate) can be used to modify properties such as swelling and mechanical properties. Monomers may also be reacted with other compounds to form macromonomers (defined above) that are then included in the polymers of the present invention. For example, HEMA can be reacted with lactide to form a HEMA-poly-lactic acid polymer (PLA/HEMA), which itself can be used as a monomer in the polymers of the present invention. In addition, the monomers themselves may be combinations of monomer units, which are then incorporated into the polymer. An example of this type of monomer is oligo(ethylene glycol) monomethyl ether methacrylate (OEGMA), which is a hydrophilic monomer composed of two hydrophilic monomers: ethylene glycol and methacrylate.

The preferred polymers of the present invention may be further modified with one or more moieties and/or functional groups. Any moiety or functional group can be used in accordance with the present invention. In some embodiments, polymers may be modified with polyethylene glycol (PEG), with a carbohydrate, and/or with acyclic polyacetals derived from polysaccharides. In addition, as discussed above, hydrophilic groups can be incorporated into monomers (and therefore polymers) to increase the water-binding capacity of the polymer.

In terms of sequence, copolymers may be block copolymers, graft copolymers, random copolymers, blends, mixtures, and/or adducts of any of the foregoing and other polymers. Typically, polymers in accordance with the present invention are organic polymers. Preferably, the polymers of the present invention are biocompatible. In some embodiments, the polymers are biodegradable. In other embodiments, the polymers are both biocompatible and biodegradable.

The preferred polymers of the present invention may also include other monomers in their structure. For example, the monomers may be polymers such as poly(vinyl alcohol) (PVA), polyesters, acrylic polymers and ionic polymers, or monomers of these.

If it is desired that the polymer be biodegradable or absorbable, one or more monomers having biodegradable linkages may be used. In the alternative, or in addition, the monomers may be chosen such that the product of the reaction between them results in a biodegradable linkage. For each approach, monomers and/or linkages may be chosen such that the resulting biodegradable polymer will degrade or be absorbed in a desired period of time, *e.g*., from about 6 h to about 6 months. Preferably, the monomers and/or linkages are selected such that, when the polymer degrades under physiological conditions, the resulting products are non-toxic.

The biodegradable linkage may be chemically or enzymatically hydrolysable or absorbable. Illustrative chemically-hydrolysable biodegradable linkages include polymers, copolymers and oligomers of glycolide, lactide, caprolactone, dioxanone, and trimethylene carbonate. Illustrative enzymatically-hydrolysable biodegradable linkages include peptidic linkages cleavable by metalloproteinases and collagenases. Additional illustrative biodegradable linkages include polymers and copolymers of poly(hydroxy acid)s, poly(orthocarbonate)s, poly(anhydride)s, poly(lactone)s, poly(aminoacid)s, poly(carbonate)s, and poly(phosphonate)s.

The chemical hydrolysation of lactide in the invention results in the increase of lower critical solution temperature (LCST) of the polymer (by decreasing the overall hydrophobicity of the polymer) and thus its bioresorptive capacity.

### Preferred polymers

The polymer preferably comprises the first monomer in an amount of from about 1 to about 15 mol%. In various embodiments, the first monomer may be present in about 1, about 2, about 3, about 4, about 5, about 6, about 7, about 8, about 9, about 10, about 11, about 12, about 13, about 14, or about 15 mol%. Preferably, the first monomer is OEGMA.

The polymer preferably comprises the second monomer in an amount of from about 5 to about 50 mol%. In various embodiments, the second monomer may be present in about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50 mol%. Preferably, the second monomer is PLA/HEMA.

The polymer preferably comprises the third monomer in an amount of up to 15 mol%. In various embodiments, the third monomer may be present in about 0, about 1, about 2, about 3, about 4, about 5, about 6, about 7, about 8, about 9, about 10, about 11, about 12, about 13, about 14, or about 15 mol%. Preferably, the third monomer is NAS.

The polymer preferably comprises the fourth monomer in an amount which makes up the remainder to 100% of the polymer composition, for example, from about 50 and about 85 mol%. In an embodiment, the mol% of the fourth monomer can be up to about 85%, preferably, about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84 or 85 mol%. Preferably, the fourth monomer is NIPAAm.

The percentages recited herein relate to the composition of the final polymer and not the feed amounts utilised when forming the polymer.

In one embodiment, the polymer preferably comprises: the first monomer in an amount of from about 1 to about 15 mol%; the second monomer in an amount of from about 5 to about 50 mol%; the third monomer in an amount of up to 15 mol%; and the fourth monomer in an amount of up to about 85 mol%.

Preferably, the first monomer is OEGMA, the second monomer is PLA/HEMA, the third monomer is NAS and the fourth monomer is NIPAAm.

In another embodiment, the polymer preferably comprises: the first monomer in an amount of about 7 mol%; the second monomer in an amount of about 30 mol%; the third monomer in an amount of about 7 mol%; and the fourth monomer in an amount of about 53 mol%.

Preferably, the first monomer is OEGMA, the second monomer is PLA/HEMA, the third monomer is NAS and the fourth monomer is NIPAAm.

In one embodiment, the polymer of the present invention is a polymer of formula (I): 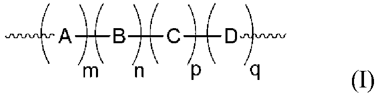 wherein
A is the first monomer (a water-binding monomer), for example, OEGMA;
B is the second monomer (a monomer that is capable of imparting mechanical properties to a tissue scaffold), for example, PLA/HEMA;
C is the third monomer (a monomer that has a functional group for binding to an NSPP), for example, NAS; and
D is the fourth monomer (a monomer that is capable of imparting phase transition characterstics to the scaffold), for example, NIPAAm.

In various embodiments, m is an integer from 1 to 20; n is an integer from 1 to 20; p is an integer from 0 to 20; and q is an integer from 1 to 20.

An exemplary polymer of the present invention is represented by Formula (Ia), below: 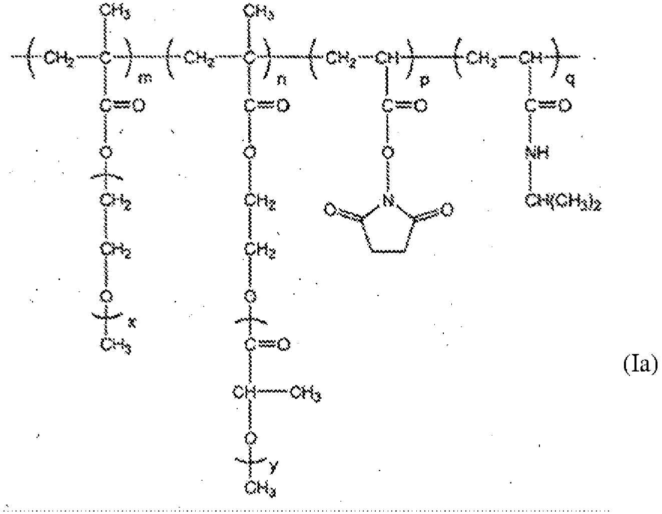 wherein A is the water-binding monomer OEGMA, B is the strengthening monomer PLA/HEMA, C is the crosslinker NAS, D is the phase transition monomer NIPAAm, and m, n and p, q, x and y are as defined above.

A person skilled in the art will be aware that the monomers A, B, C and D may be present in the polymer in any order, provided that the required water-binding, strengthening and/or cross-linking capabilities are achieved.

It has also been discovered that some monomers, such as PLA/HEMA, polyesters such as poly(lactic acid), poly(caprolactone), poly(glycolide), and their random copolymers (*e.g*., poly(glycolide-co-lactide) and poly(glycolide-co- caprolactone) and other biodegradable and biocompatible polymers, can elevate the LCST of the preferred polymer used in the present invention during degradation of biodegradable segments (*e.g*., PLA) *in vivo*, leading to bioresorption of the polymer. This provides the additional advantage that the polymers used in the present invention may be designed so as to be biodegradable *in vivo*.

The overall size of the preferred polymer used in the present invention may differ, depending on factors such as the types of monomers that are incorporated into the polymer, the type of NSPP that is sought to be used to form the scaffold, and the conditions under which the protein is to be coupled to the polymer. However, in general, the preferred polymer used in the present invention may be a molecule of about 1 to about 100 kDa, about 5 to about 60 kDa, or about 30 kDa. In various embodiments, the polymer of the present invention may be a molecule of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or about 100 kDa.

### PNPHO

A preferred polymer of the present invention is Poly(NIPAAm-*co*-NAS-*co-*(PLA/HEMA)-*co*-OEGMA), *i.e*., "PNPHO". The polymer PNPHO preferably comprises OEGMA in an amount of from about 1 and about 15 mol%, PLA/HEMA in an amount of from about 5 and about 50 mol%, NAS in an amount of up to 15 mol%, and NIPAAm in an amount which makes up the remainder to 100% of the polymer composition, for example, from about 50 to about 85 mol%.

The percentages recited herein relate to the composition of the final polymer and not the feed amounts utilised when forming the polymer.

A preferred form of the polymer PNPHO for use in the present application is a polymer of Formula (Ia), as drawn above. In addition, x is in the range of 1-1000 and y is in the range of 1-1000 and m, n, p, and q are in the range of 1-20.

A person skilled in the art will be aware that the monomers A, B, C and D may be present in the polymer in any order, provided that the required water-binding, strengthening and/or cross-linking capabilities are achieved.

### PPHO

Another preferred polymer of the present invention is Poly(NIPAAm-*co-*(PLA/HEMA)-*co*-OEGMA), *i.e*., "PPHO". The polymer PPHO preferably comprises OEGMA in an amount of from about 1 and about 15 mol%, PLA/HEMA in an amount of from about 5 and about 50 mol%, and NIPAAm in an amount which makes up the remainder to 100% of the polymer composition, for example, from about 50 to about 85 mol%. In preferred embodiments, PPHO comprises OEGMA in about 1, 2, 3, 4, 5, 6, 7, 8, 9, 10 ,11, 12, 13, 14 or about 15 mol% and/or PLA/HEMA in about 15, 16 ,17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or about 50 mol% and/or NIPAAM in an amount of about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84 or about 85 mol%.

The percentages recited herein relate to the composition of the final polymer and not the feed amounts utilised when forming the polymer.

A preferred form of the polymer PPHO for use in the present application is a polymer of Formula (II), as drawn below. In addition, x is in the range of 1-1000 and y is in the range of 1-1000 and m, n, and q are in the range of 1-20.

A person skilled in the art will be aware that the monomers A, B, and D may be present in the polymer in any order, provided that the required water-binding, strengthening and/or cross-linking capabilities are achieved.

### Synthesis of polymers

A person skilled in the art will be aware of suitable methods of synthesising the preferred polymers used in the present invention. These include methods such as ring-opening polymerisation, addition polymerisation (including free radical polymerisation) and condensation polymerisation.

The formation of the preferred polymers, PNPHO and PPHO, is described in the examples below.

### Compositions for forming scaffolds

The present invention also relates to a polymer for forming a tissue scaffold, the polymer comprising a first water-binding monomer; a second monomer that imparts mechanical properties; an optional third monomer that is an NSPP-binding monomer; and a fourth monomer capable of imparting phase transition characteristics to the scaffold

The term "composition", as used herein, refers to a solid or liquid composition containing the components mentioned above. In some embodiments, other components such as pharmaceutically-acceptable excipients and biologically active agents (*e.g*., drugs, vitamins and minerals), to assist in repair and/or re-generation of the target tissue, and/or to provide a method of achieving targeted delivery of biologically active compounds, may also be included in the compositions of the present invention.

### Excipients and biologically-active agents

Pharmaceutically-acceptable excipients may be included in the compositions and/or scaffolds of the present invention, and include any and all solvents, dispersion media, inert diluents, or other liquid vehicles, dispersion or suspension aids, granulating agents, surface active agents, disintegrating agents, isotonic agents, thickening or emulsifying agents, preservatives, binding agents, lubricants, buffering agents, oils, and the like, as suited to the particular dosage form desired. Remington (Gennaro, A. R., Remington: The Science and Practice of Pharmacy, 21st Ed (2006) Lippincott Williams & Wilkins) discloses various excipients used in formulating pharmaceutical compositions and known techniques for the preparation thereof. Except insofar as any conventional excipient is incompatible with a substance or its derivatives, such as by producing any undesirable biological effect or otherwise interacting in a deleterious manner with any other component(s) of the pharmaceutical composition, its use is contemplated to be within the scope of this invention.

Excipients such as colouring agents, coating agents, sweetening, flavouring, and perfuming agents can be present in the composition, according to the judgment of the formulator.

Biologically active agents or drug compounds that may be added to the composition and/or scaffolds of the present invention include proteins, glycosaminoglycans, carbohydrates, nucleic acids and inorganic and organic biologically active compounds, such as enzymes, antibiotics, anti-neoplastic agents, local anaesthetics, hormones, angiogenic agents, anti-angiogenic agents, growth factors (*e.g*., insulin-like growth factor-1 (IGF-1), basic fibroblast growth factor (bFGF) and transforming growth factor-b (TGFb)), antibodies, neurotransmitters, psychoactive drugs, anticancer drugs, chemotherapeutic drugs, drugs affecting reproductive organs, genes, and oligonucleotides.

A composition containing components such excipients and/or biologically active agents can be produced by combining a polymer of the present invention with an NSPP, combining this with one or more other components and then freeze-drying the resulting composition. This leads to a ready to use tissue scaffold.

The amount of polymer, NSPP and biologically active agent present in the composition will necessarily depend upon the particular drug and the condition to be treated. A person skilled in the art will be aware of appropriate agents and amounts to use to treat the condition.

### Natural or synthetic peptide or protein

The NSPP can be important because it provides additional mechanical properties (such as strength and resilience) to the scaffold, as well as providing, at the repair site, an environment that mimics the natural environment, thereby assisting in tissue repair and regeneration.

It is important that the NSPP contains side chains or other functional groups that are exposed to enable reaction with the functional group of the NSPP -binding monomer(s), thereby binding the NSPP to the polymer through the NSPP-binding monomer(s). Examples of suitable side chains include glutamic acid or lysyl side chains.

The present invention also contemplates the use of variants of the NSPPs, for example species variants or polymorphic variants. The present invention is intended to cover all functionally active variants of the NSPPs that exhibit the same activity. This also includes apo- and haloforms of the NSPPs, post-translationally modified forms, as well as glycosylated or deglycosylated derivatives. Such functionally active fragments and variants include, for example, those having conservative amino acid substitutions.

Preferably, the NSPP(s) for use in the present invention will be obtained from recombinant sources, although they can also be extracted from natural sources or synthesised.

### Scaffolds

The present invention also relates to a tissue scaffold comprising a polymer according to the invention and an optional NSPP, wherein the polymer comprises: a first water-binding monomer; a second monomer that imparts mechanical properties; an optional third monomer that is an NSPP-binding monomer, comprising a functional group that is capable of binding to the NSPP; and a fourth monomer capable of imparting phase transition characteristics to the scaffold, wherein the scaffold forms a malleable structure upon hydration. Preferably, the first monomer is OEGMA, the second monomer is PLA/HEMA, the third monomer is NAS and the fourth monomer is NIPAAm.

In one embodiment, the scaffold includes a polymer having a monomer described above for ensuring post-administration stability of the scaffold. Specifically, the LCST of the scaffolds is below a body temperature, or about 37 °C to avoid formation of unstable composite or dissolutio*n in vivo*. One example of a monomer useful for this purpose is NIPAAm.

In another embodiment, the amount of the first monomer in the polymer do not exceed about 15 mol% to ensure that the LCST of the scaffolds is below a body temperature, or about 37 °C. One example of a monomer useful for this purpose is OEGMA.

In yet another embodiment, the amount of the second monomer in the polymer can be as high as 50 mol% to act as a backbone of the polymer.

### Cells

The scaffold of the present invention may also include cells to assist in repair and/or regeneration of the target tissue.

In general, cells to be used in accordance with the present invention are any types of cells. The cells should be viable when embedded with the scaffolds of the present invention.

In some embodiments, cells that can be embedded to the scaffolds in accordance with the present invention include, but are not limited to, mammalian cells (*e.g*., human cells, primate cells, mammalian cells, rodent cells, *etc*.), avian cells, fish cells, insect cells, plant cells, fungal cells, bacterial cells, and hybrid cells. In some embodiments, exemplary cells that can be embedded to the scaffold include stem cells, totipotent cells, pluripotent cells, and/or embryonic stem cells.

In some embodiments, exemplary cells that can be embedded to the scaffolds in accordance with the present invention include, but are not limited to, primary cells and/or cell lines from any tissue. For example, cardiomyocytes, myocytes, hepatocytes, keratinocytes, melanocytes, neurons, astrocytes, embryonic stem cells, adult stem cells, hematopoietic stem cells, hematopoietic cells (*e.g*., monocytes, neutrophils, macrophages, *etc*.), ameloblasts, fibroblasts, chondrocytes, osteoblasts, osteoclasts, neurons, sperm cells, egg cells, liver cells, epithelial cells from lung, epithelial cells from gut, epithelial cells from intestine, liver, epithelial cells from skin, etc, and/or hybrids thereof, may be embedded to scaffolds in accordance with the present invention.

Exemplary mammalian cells that can be embedded to scaffolds in accordance with the present invention include, but are not limited to, Chinese hamster ovary (CHO) cells, HeLa cells, Madin-Darby canine kidney (MDCK) cells, baby hamster kidney (BHK cells), NSO cells, MCF-7 cells, MDA-MB-438 cells, U87 cells, A172 cells, HL60 cells, A549 cells, SP10 cells, DOX cells, DG44 cells, HEK 293 cells, SHSY5Y, Jurkat cells, BCP-1 cells, COS cells, Vero cells, GH3 cells, 9L cells, 3T3 cells, MC3T3 cells, C3H-10T1/2 cells, NIH-3T3 cells, and C6/36 cells.

In some embodiments, it is desirable that cells are evenly distributed throughout a scaffold. Even distribution can help provide more uniform tissue-like scaffolds that provide a more uniform environment for encapsulated cells. In some embodiments, cells are located on the surface of a scaffold. In some embodiments, cells are located in the interior of a scaffold. In some embodiments, cells are layered within a scaffold. In some embodiments, the scaffold contains different cell types.

In some embodiments, the conditions under which cells are embedded to scaffolds are altered in order to maximise cell viability. In some embodiments, for example, cell viability increases with lower polymer concentrations. In some embodiments, cells located at the periphery of a hydrogel tend to have decreased viability relative to cells that are fully-encapsulated within the hydrogel. In some embodiments, conditions (*e.g*., pH, ionic strength, nutrient availability, temperature, oxygen availability, osmolarity, *etc*.) of the surrounding environment may need to be regulated and/or altered to maximise cell viability.

In some embodiments, cell viability can be measured by monitoring one of many indicators of cell viability. In some embodiments, indicators of cell viability include, but are not limited to, intracellular esterase activity, plasma membrane integrity, metabolic activity, gene expression, and protein expression. To give but one example, when cells are exposed to a fluorogenic esterase substrate (*e.g*., calcein AM), live cells fluoresce green as a result of intracellular esterase activity that hydrolyses the esterase substrate to a green fluorescent product. To give another example, when cells are exposed to a fluorescent nucleic acid stain (*e.g*., ethidium homodimer-1), dead cells fluoresce red because their plasma membranes are compromised and, therefore, permeable to the high-affinity nucleic acid stain.

In general, the number of cells in a composition is an amount that allows for the formation of hydrogels in accordance with the present invention. In some embodiments, the amount of cells that is suitable for forming hydrogels in accordance with the present invention ranges between about 0.1% w/w and about 80% w/w, between about 1.0% w/w and about 50% w/w, between about 1.0% w/w and about 40% w/w, between about 1.0% w/w and about 30% w/w, between about 1.0% w/w and about 20% w/w, between about 1.0% w/w and about 10% w/w, between about 5.0% w/w and about 20% w/w, or between about 5.0% w/w and about 10% w/w.

In some embodiments, the amount of cells in a composition that is suitable for forming hydrogels in accordance with the present invention is approximately 5% w/w. In some embodiments, the concentration of cells in a precursor solution that is suitable for forming hydrogels in accordance with the invention ranges between about 10 and about 1×10⁸ cells/mL, between about 100 and about 1×10⁷ cells/mL, between about 1×10³ and about 1×10⁶ cells/mL, or between about 1×10⁴ and about 1×10⁵ cells/mL. In some embodiments, a single hydrogel comprises a population of identical cells and/or cell types. In some embodiments, a single hydrogel comprises a population of cells and/or cell types that are not identical.

In some embodiments, a single hydrogel may comprise at least two different types of cells. In some embodiments, a single hydrogel may comprise 3, 4, 5, 10, or more types of cells. To give but one example, in some embodiments, a single hydrogel may comprise only embryonic stem cells. In some embodiments, a single hydrogel may comprise both embryonic stem cells and hematopoietic stem cells.

### Media

Any of a variety of cell culture media, including complex media and/or serum-free culture media, that are capable of supporting growth of the one or more cell types or cell lines may be used to grow and/or maintain cells. Typically, a cell culture medium contains a buffer, salts, energy source, amino acids (*e.g*., natural amino acids, non-natural amino acids, *etc*.), vitamins, and/or trace elements. Cell culture media may optionally contain a variety of other ingredients, including but not limited to, carbon sources (*e.g*., natural sugars, non-natural sugars, *etc*.), cofactors, lipids, sugars, nucleosides, animal-derived components, hydrolysates, hormones, growth factors, surfactants, indicators, minerals, activators of specific enzymes, activators inhibitors of specific enzymes, enzymes, organics, and/or small molecule metabolites.

Cell culture media suitable for use in accordance with the present invention are commercially available from a variety of sources, *e.g*., ATCC (Manassas, VA.). In certain embodiments, one or more of the following media are used to grow cells: RPMI-1640 Medium, Dulbecco's Modified Eagle's Medium, Minimum Essential Medium Eagle, F-12K Medium, Iscove's Modified Dulbecco's Medium.

Those skilled in the art will recognise that the cells listed herein represent an exemplary, not comprehensive, list of cells that can be encapsulated within a precursor solution in accordance with the present invention.

### Applications

The present invention aims to provide a scaffold that supports the natural healing of damaged tissue without inducing any specific tissue-formation. It is aimed to use the invention to enable immobilisation of one/two or more tissues to support tissue regeneration and/or tissue interface integration with minimal foreign body reaction.

Preferably, the tissue scaffold of the invention is to be hydrated intraoperatively with saline, a patient's own blood, platelet reach plasma (PRP), platelet reach fibrin and/or other autologous blood/cell/ tissue products to form malleable scaffolds. The resulting scaffolds can be administered arthroscopically or through open surgical intervention. In contact with body tissue, the scaffold is adhesive, and it is suturable which enables immobilisation of one or more tissues to support tissue regeneration and/or tissue interface integration.

The scaffolds of the present invention preferably can be manufactured by using different processing methods.

The scaffolds of the present invention preferably negate the criticality of water solubility.

The scaffolds of the present invention preferably can be tuned to alter their micro-environment to address the different requirements.

The scaffolds of the present invention preferably bind to different hydrophilic and hydrophobic drugs.

The scaffolds of the present invention are preferably non-adhesive to gloves for effective manipulation.

The scaffolds of the present invention are preferably malleable, which allows effective delivery to the site, arthroscopically or via open surgical intervention.

The scaffolds of the present invention are preferably adhesive to the treatment site and the scaffolds of the present invention are preferably suturable for internal fixation.

The present invention has been developed for use in skin grafting to immobilise the grafter tissue, repair of partial or full thickness rotator cuff tears, tendon healing in total shoulder arthroplasty, anterior cruciate ligament (ACL) and/or other ligament/tendon tears.

### Kits

The invention provides a variety of kits comprising one or more of the scaffolds and/or polymers of the present invention. For example, in an aspect, the invention provides a kit comprising a scaffold and/or polymers and instructions for use. A kit may comprise multiple different scaffolds and/or polymers. A kit may optionally comprise cells, NSPPs, biologically-active compounds, and the like. A kit may comprise any of a number of additional components or reagents in any combination. All of the various combinations are not set forth explicitly, but each combination is included in the scope of the invention.

### PNPHO/PPHO compositions

PNPHO was synthesised in accordance with the synthetic procedure set forth generally in the Applicant's prior publication, WO 2013/091001. PPHO was synthesised in a similar manner, save for the addition of NAS.

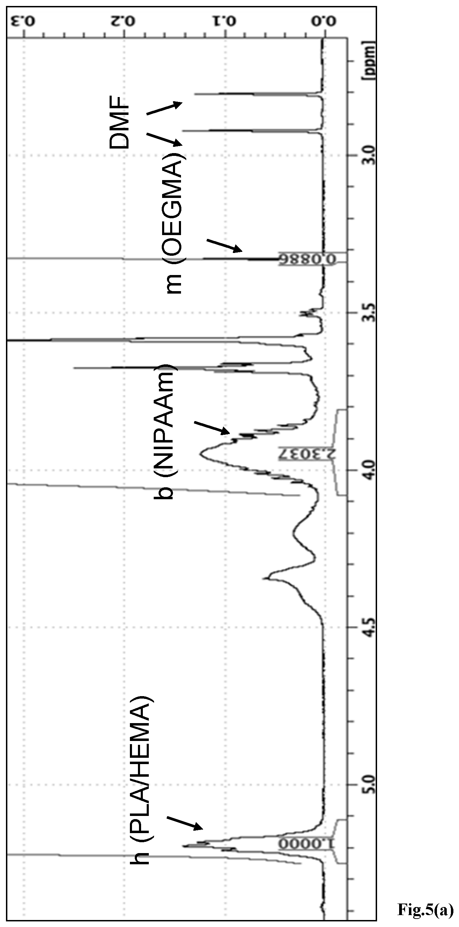

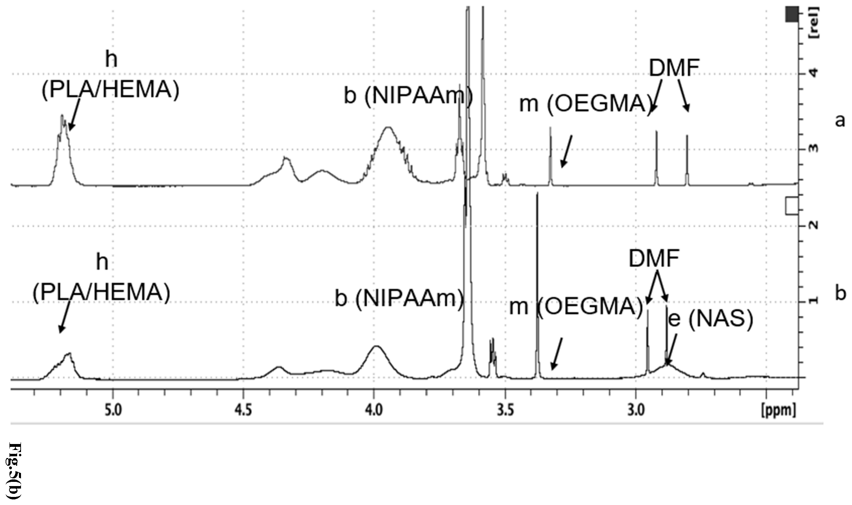

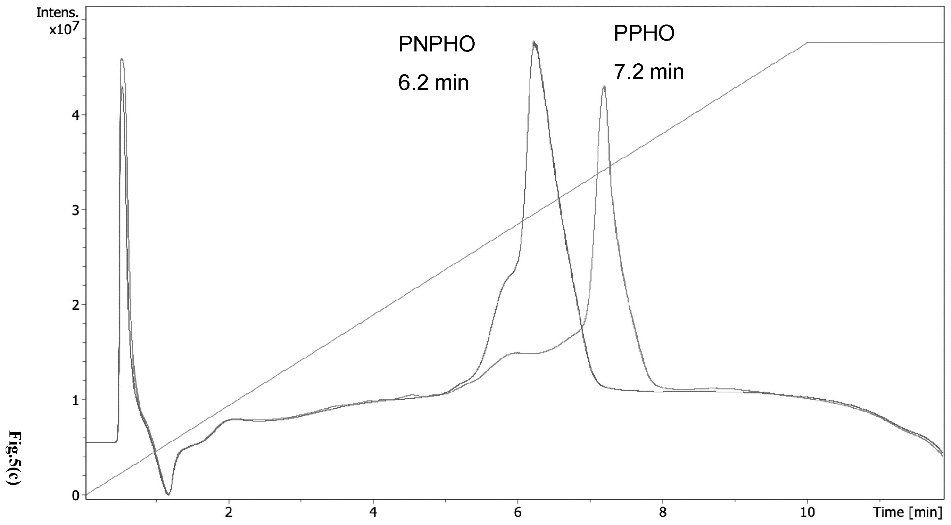

The synthesis of PNPHO copolymers was confirmed with ¹H NMR spectra with evidence of proton peaks for each monomer, consistent with those shown in WO 2013/091001. Similarly, the synthesis of PPHO was confirmed via ¹H NMR spectroscopy with evidence of proton peaks for each monomer, as shown in Figure 5(a). Comparative NMR spectra of PNPHO and PPHO are shown in Figure 5(b). As shown in Figure 5(c), HPLC chromatograms at 230 nm show PNPHO eluting at 6.2 min and PPHO at 7.2 min. The difference in the elution times showed that PPHO elutes at higher percentage acetonitrile compared to PNPHO, therefore confirming the hydrophobicity of PPHO in comparison with

### PNPHO.

Characteristic proton peaks were detected for NIPAAm (a and b), NAS (e), PLA/HEMA (f, h, k), and OEGMA (m and n). The final composition of copolymer was calculated based on the integration of these peaks from each monomer as for NIPAAm (a), NAS(e/2-f), PLA/HEMA (h), and OEGMA (n/2). In this study copolymer is denoted as PNPHO and the subscript is added that corresponds to PLA/HEMA (lactate length) to OEGMA molar ratios. For example PNPHO₈₍₆₎₃ stands for the copolymer synthesized with 8 mol% PLA/HEMA with lactate length of 6, and 3 mol% OEGMA. Various copolymers were produced.

### Manufacturing of a tissue scaffold from PNPHO/PPHO

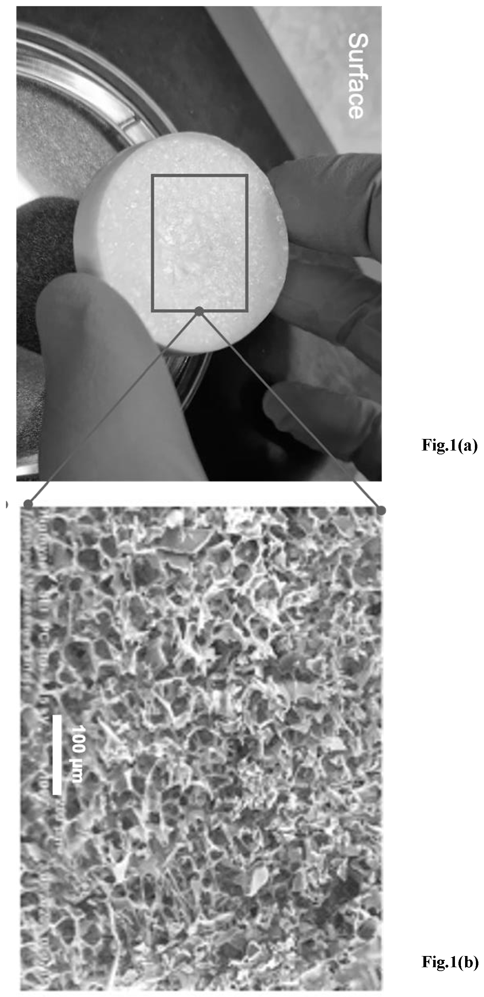

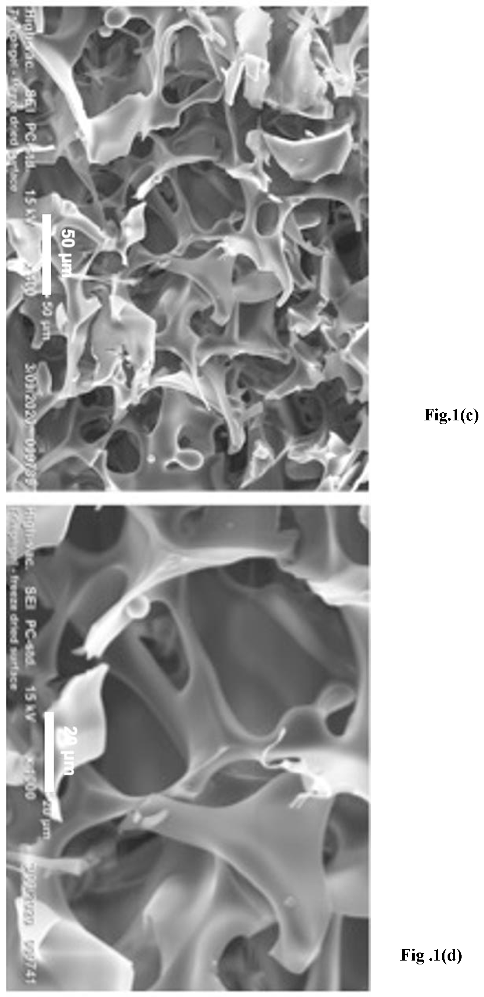

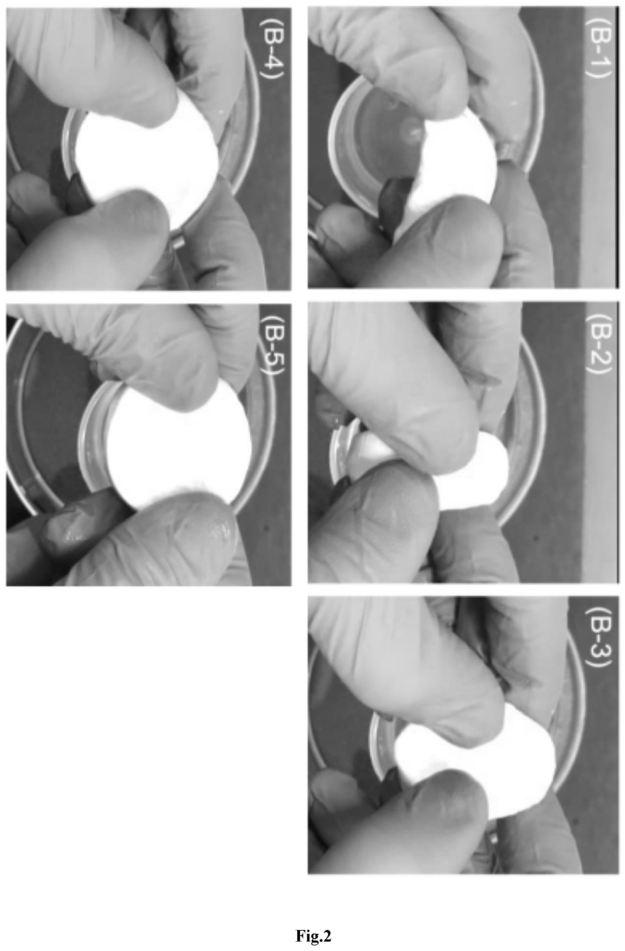

The tissue scaffolds of the present invention can be manufactured from PNPHO/PPHO solution using different methods. The resulting PNPHO/PPHO scaffolds and their morphology are shown in Figure 1. Upon hydration, the scaffolds can be shaped and if required for arthroscopic delivery, as shown in Figure 2. The product is amenable to suturing and surgical stapling. The product is not adhesive to gloves which allow effective manipulation.

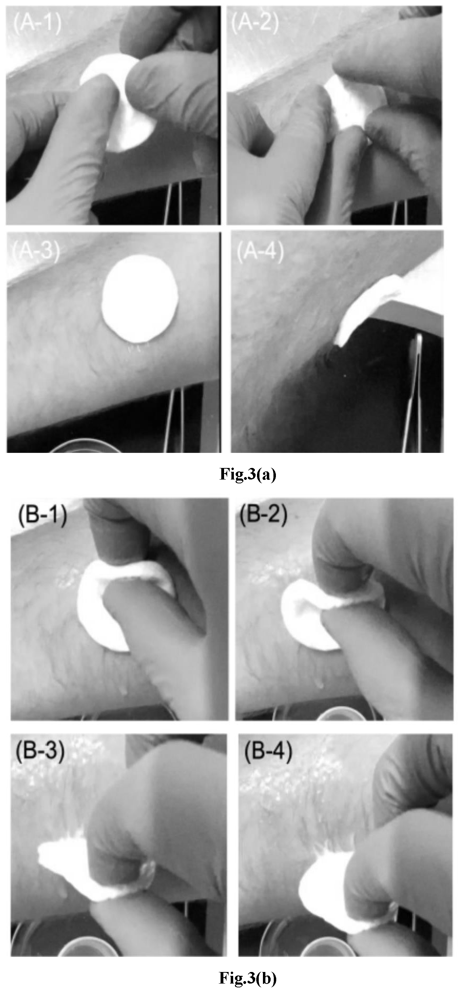

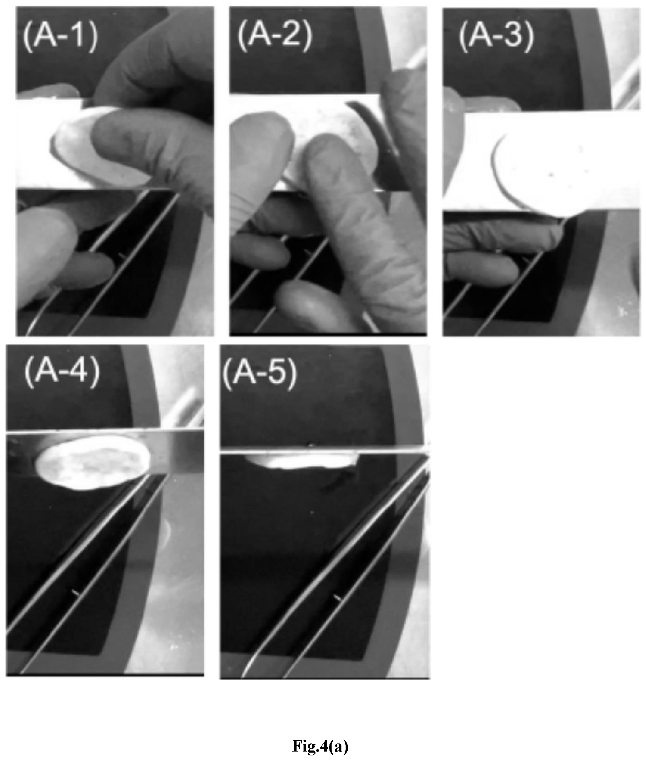

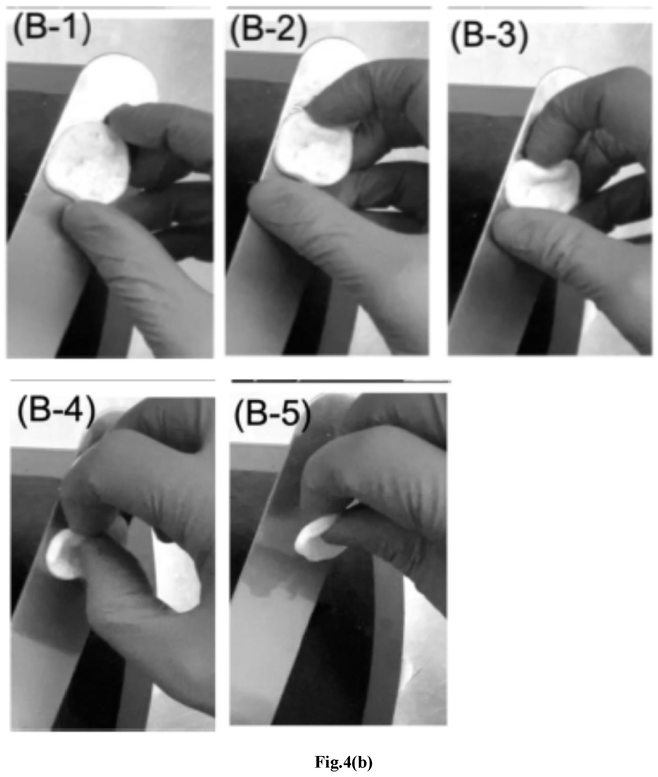

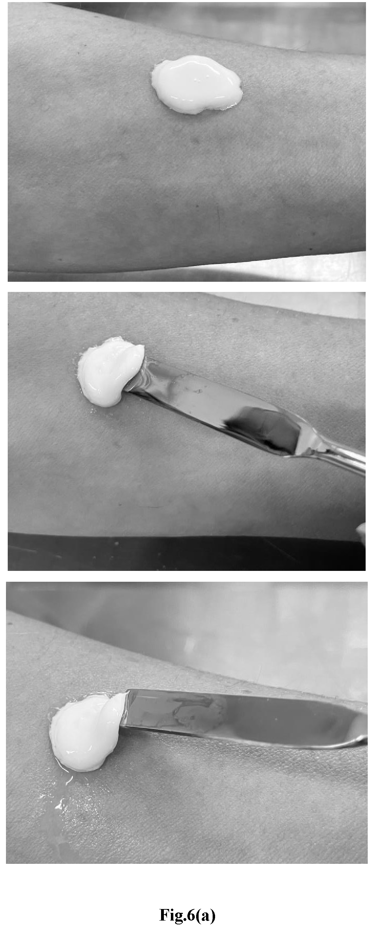

In contact with different tissues (as shown in Figures 3, 4 and 6), the product is adhesive which allows *in vivo* immobilisation of one or more tissues with/without suturing/internal stabilisation.

In general, the PHPHO/PPHO scaffolds of the present invention can be prepared by freeze drying of a water-soluble composition, wherein PNPHO/PPHO solution with or without peptide/protein components are dissolved in buffered solutions; different drugs/moieties (antibiotics, growth factors, live viruses) can be added to the solution for final product embedding; the resulting solution is freeze dried and the morphology of the composites can be altered by adjusting the freeze drying parameters and the concentration of PNPHO polymer; and the final product is in the form of ready to use scaffolds with or without embedded biological moieties.

### Exemplary Method 1

A first method is employed where the polymer is water soluble. It involves dissolving an aqueous solution of the polymer in a buffer solution; and freeze-drying the resultant solution, wherein the morphology of the scaffold is altered by adjusting freeze-drying parameters and concentration of the polymer.

### Exemplary Method 2

A second method is employed where the polymer is hydrophobic (*e.g*., PPHO). It involves dissolving the polymer in an organic solvent to obtain a solution; precipitating the solution in water as an anti-solvent to obtain a gel; and freeze-drying the gel, wherein the morphology of the scaffold is altered by adjusting freeze-drying parameters.

Other preparatory methods may involve porogen leaching and/or gas foaming.

### Exemplary Method 3

A method is employed to produce injectable or sprayable scaffold. It involves dissolving a PPHO polymer in aqueous solutions, autologous blood, cell or tissue products, or a combination thereof.

### Scaffold fabrication

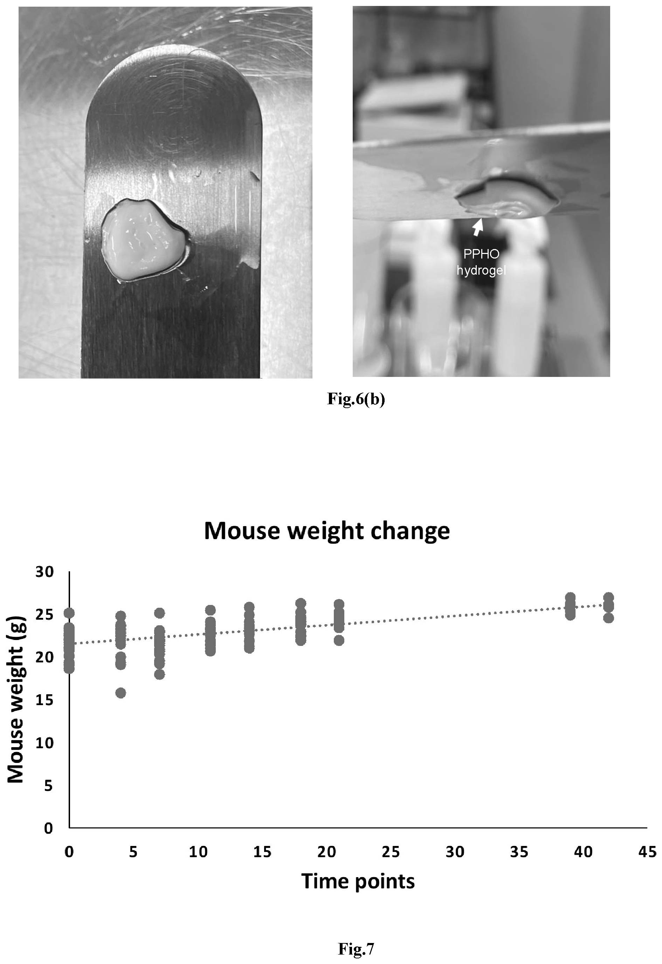

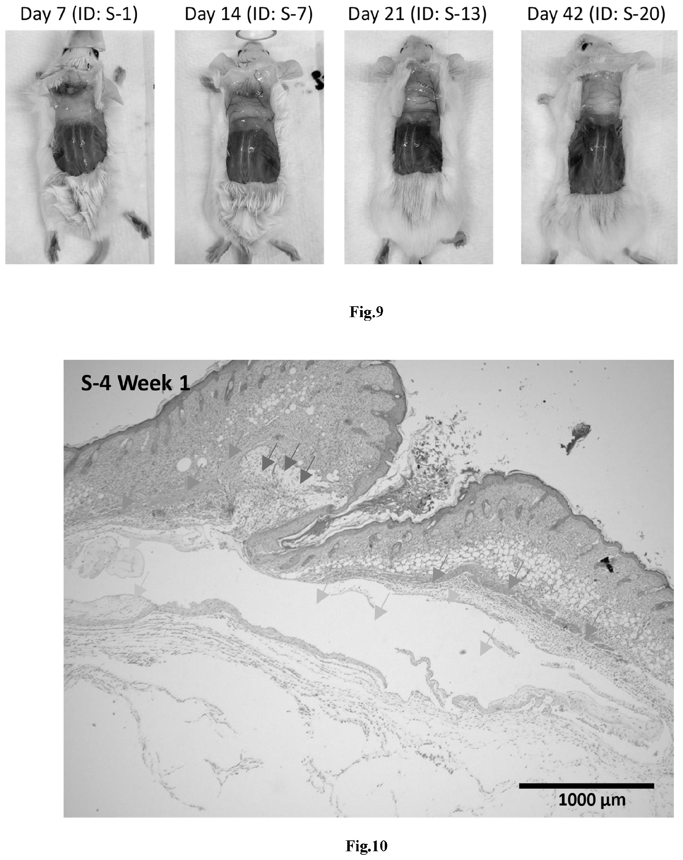

According to the invention the scaffolds are fabricated either by freeze drying of PNPHO and/or PPHO to form porous films as previously outlined and shown in Figures 7 and 10. In addition, the scaffolds can be fabricated by press compression of PNPHO and/or PPHO powders. SEM imaging of both configurations showed similar microstructure and topography, however, the scaffold fabricated using press compression was used for *in vivo* biocompatibility analyses.

The purpose of this study was to investigate the biodegradability of lyophilised TM scaffolds in a murine model. 24 male Balb/c mice, approximately 10-weeks old were subcutaneously implanted with lyophilised PNPHO and PPHO scaffolds. Ultrasound measurements were performed weekly to determine presence and volume *in situ*. Animals were sacrificed at day 7, day 14, day 21 and day 42 post-implantation. Biopsies of the implantation sites were harvested and fixed in 10% formalin, embedded in paraffin, sectioned, and stained with H&E for histological analysis.

### Surgical procedure and product preparation

PNPHO and PPHO scaffolds were used. Prior to the surgery, the mice were anesthetised using 3% isoflurane. General anesthesia was noted by a lack of response to a toe pinch.). A 1 cm excision was created on the mouse dorsal skin and the scaffolds were inserted subcutaneously. Then the incision wound was closed by sutures and cleaned if necessary.

### Handling of material

Prior to implantation, the scaffolds were hydrated in 2 mL of warm water at around 37 °C for at least 3 minutes. The scaffolds were found to be cohesive and mouldable. The scaffolds could be easily picked up with forceps and delivered into the pouch created on the dorsum of the mouse with ease and convenience.

### Recovery

All the experimental mice recovered within 5 minutes from anaesthesia. The mice were recorded as healthy with no signs of discomfort or stress. The procedure did not impact the mouse mobility and no scaffold was detected coming out from the incision wound after it was closed by sutures. The scaffolds seemed to remain solid in the mouse body for a time.

### Animal health and weight

No abnormality in health conditions (and no significant weight loss) was found in all mice over the experimental period with exception of mouse ID "S-21". This mouse displayed significant weight loss on day 4 post-operatively, without any other abnormal signs or signs of distress; the mouse was behaving normally, did not have any wounds or bite marks or display any hair loss. As all other parameters were normal, the mouse was maintained for further observation. Its weight increased steadily on day 7 and returned to the normal level on day 11.

### Macroscopic observations and ultrasound analysis

The mice were scanned with high-frequency ultrasound to examine the volume of scaffolds after implantation. The ultrasound was performed immediately after implantation and at time points day 0, day 4, day 7, day 11 and day 14. Immediately after the procedure, the scaffolds were visibly detected and palpable, however, ultrasound imaging did not detect the scaffold, likely due to its solid structure.

### Biopsy and autopsy

At time points, day 7, day 14, day 21 and day 42, groups of mice were sacrificed, and the scaffold presence was macroscopically scored. The skin biopsies were collected and stored in 10% formalin for histological analysis. The scaffolds were integrated within the host environment at all time points of the harvest. The panniculus carnosus layer was found to be more adhesive to the underlying structures compared to historical control group and experience of this anatomical layer in mice. The skin was more adhesive to the subcutaneous fascia and musculature. The scaffolds are believed to be integrated within the tissue layers of the underlying tissue.

### Histological analysis

The collected skin biopsies of the implantation sites were processed and stained with H&E to analyse TM scaffold presence, cell infiltration, inflammatory response and angiogenesis.

The histological evaluations of the sites after 1 week in Figure 10 showed the structure of scaffolds with cell infiltration (indicated by blue arrows). The scaffolds implanted in the mouse dorsum should sit underneath panniculus carnosus layer (indicated by orange arrows). The underlying area shows a large space with the scaffolds with cell infiltration (indicated by yellow arrows).

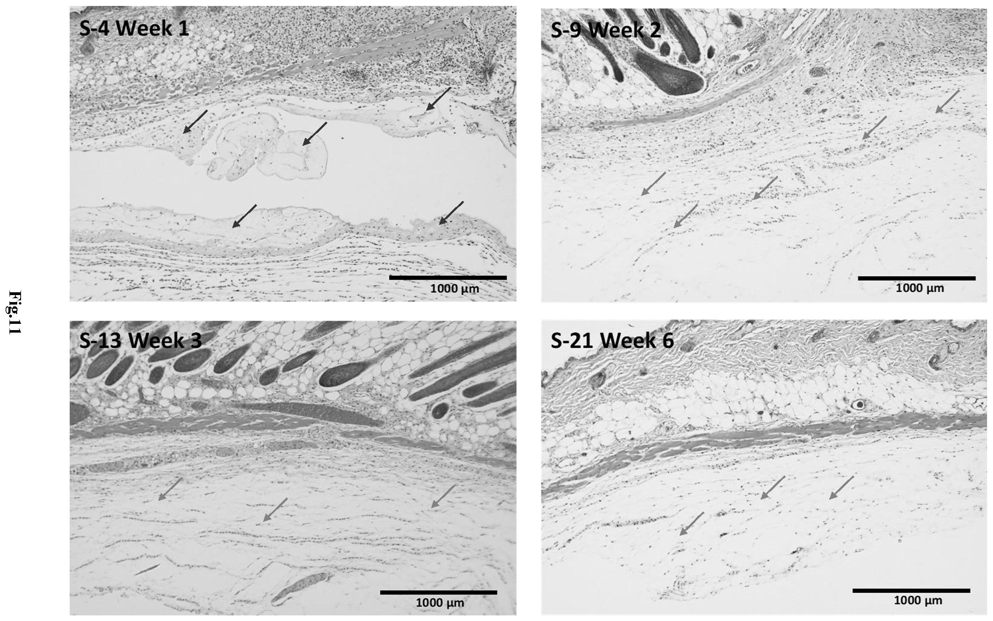

The results in Figure 11 showed that at week 1, scaffolds present in histology with infiltration of a number of cells (indicated by red arrows). In weeks 2, 3, and 6, the remaining scaffold could be detected histologically, while the host tissues have loose connective tissue structures, likely fascia (indicated by green arrows). It is important to note that both PNPHO and PPHO are highly soluble in ethanol and therefore are removed during the processing steps. Nonetheless, there was no sign of scaffold encapsulation, fibrosis and fibrotic tissue formation at around the scaffolds, confirming the biocompatibility of the products.

The mild cell infiltration observed after 1 week of implantation were consistent with the early stages of wound healing and foreign body reactions. No obvious multi-nucleated giant cells were detected in the area where the scaffold was present. Large areas of cell infiltration were observed surrounding sutures, which is expected and considered a typical foreign body reaction in suture biodegradation. There was no cell inflammatory response observed in week 2, week 3, and week 6.

### Other notes

The scaffolds used in the experiment could be handled in a manner that allowed for easy implantation and convenient administration after being hydrated. The healthy recovery of all experimental mice and the absence of abnormal signs of discomfort or stress immediately after the procedure are also indicative of the biocompatibility with *in vivo* life.

The detection of solid scaffolds within the mouse body immediately after implantation suggests that the scaffolds remained *in situ* and were not easily dislodged or degraded within the body. The absence of significant weight loss in all mice, also indicates the overall health and stability of the animals during the experimental period. The results of the procedure suggest that the scaffolds used were effective in their intended purpose and that the implantation procedure was successful overall. Macroscopically, the investigators were easily able to identify the tablet-shaped scaffold subcutaneously immediately following the implantation procedure. The scaffold did not turn into a liquid state but remained solid.

### Industrial Applicability

It will be appreciated that the present invention finds ready applicability in the surgical and biomedical fields. The inventive PNPHO or PPHO scaffold can be used at any anatomical site in which internal immobilisation of one or more tissue/s is required to support healing. These include but are not limited to skin grafting to immobilise the grafter tissue, repair of partial or full thickness rotator cuff tears, tendon healing in total shoulder arthroplasty, anterior cruciate ligament (ACL) and/or other ligament/tendon tears and the like.

## Claims

1. A polymer for forming a tissue scaffold, the polymer comprising:
oligo(ethylene glycol) monomethyl ether methacrylate (OEGMA) in an amount of about 1-15 mol%;
hydroxyethyl methacrylate poly(lactic acid) (PLA/HEMA) in an amount of about 5-50 mol%; and
N-isopropylacrylamide (NIPAAm) in an amount comprising the remainder of the polymer,
wherein the polymer is capable of forming a tissue scaffold having a malleable structure upon hydration.

2. The polymer of claim 1, wherein the polymer is poly(N-isopropylacrylamide-co-(polylactide/2-hydroxyethyl methacrylate)-co-(oligo(ethylene glycol) monomethyl ether methacrylate)) (PPHO).

3. The polymer of any one of the preceding claims, having a lower critical solution temperature below 37 °C.

4. The polymer of any one of the preceding claims, wherein degradation of PLA moieties increases the lower critical solution temperature of the polymer.

5. A tissue scaffold comprising the polymer of any preceding claim, wherein the scaffold forms a malleable structure upon hydration.

6. The tissue scaffold of claim 5, wherein the scaffold is adhesive to tissue.

7. The tissue scaffold of claim 5 or claim 6, wherein the scaffold is non-adhesive to surgical gloves.

8. The tissue scaffold of any one of claims 5 to 7, wherein the scaffold is suturable or staplable.

9. The tissue scaffold of any one of claims 5 to 8, wherein the scaffold is adapted to be hydrated with saline, an aqueous solution, blood, platelet-rich plasma, platelet-rich fibrin, bone marrow aspirate, or another blood-, cell- or tissue-derived product.

10. A method of making a tissue scaffold comprising:
dissolving the polymer of any one of claims 1 to 4 in an organic solvent;
precipitating the solution in water as an anti-solvent to obtain a gel; and
freeze-drying the gel to form a tissue scaffold.

11. The method of claim 10, wherein the morphology of the scaffold is altered by adjusting freeze-drying parameters.

12. The method of claim 10 or claim 11, wherein the organic solvent is miscible with water.

13. A tissue scaffold according to any one of claims 5 to 9 for use in tissue repair and/or tissue regeneration.

14. The tissue scaffold for use according to claim 13, wherein the tissue repair and/or tissue regeneration comprises skin graft immobilisation.

15. The tissue scaffold for use according to claim 13, wherein the tissue repair and/or tissue regeneration comprises tendon repair, ligament repair, or rotator cuff repair.
